# EUROPÄISCHE PATENTSCHRIFT

(11) **EP 2 055 294 B1**
(45) Veröffentlichungstag und Bekanntmachung des Hinweises auf die Patenterteilung: **11.07.2012**
(21) Anmeldenummer: 08162537.8
(22) Anmeldetag: 18.08.2008
(51) Int. Cl.: A61K 8/19, A61K 8/81, A61Q 5/02, A61Q 5/12

(54) **Haarbehandlungsmittel enthaltend ein vernetztes Polymer auf Acrylatbasis**
Hair treatment agent comprising a crosslinked acrylic polymer
Produit de traitement capillaire comprenant un polymère acrylique reticulé

(30) Priorität: 17.10.2007 DE 102007050371
(43) Veröffentlichungstag der Anmeldung: 06.05.2009
(73) Patentinhaber: Beiersdorf AG, 20245 Hamburg (DE)
(72) Erfinder: Neuhoff, Henrike, 22359 Hamburg (DE); Wilken, Maren, 22848 Norderstedt (DE)

(56) Entgegenhaltungen:
- EP-A- 0 463 780
- EP-A- 1 348 420
- EP-A- 1 723 945
- EP-A- 2 020 223
- GB-A- 1 418 024
- US-A- 5 344 643
- US-A- 6 106 816
- "2-in-1 Conditioning Shampoo" INTERNET CITATION, [Online] 14. November 2006 (2006-11-14), XP002521545 Gefunden im Internet: URL:http://web.archive.org/web/20061114133 318/http://www.personalcare.noveon.com/for mulas/SH-0020(EU).pdf> [gefunden am 2009-03-27]
- "ULTRA MILD CONDITIONING SHAMPOO WITH SUSPENDED MICA USING CARBOPOL ETD 2020 POLYMER" INTERNET CITATION, [Online] XP002305256 Gefunden im Internet: URL:http://www.personalcare.noveoninc.com/ formulations/EU0010.pdf> [gefunden am 2004-11-11]

## Beschreibung

Die Erfindung betrifft Haarshampoos enthaltend Tensid und vernetzte Polymere auf Acrylatbasis.

Haarshampoos sind hinlänglich bekannt.

Haarglanz ist für den Verbraucher eines der wichtigsten Produktanforderungen im Hair Care Bereich, denn Schönheit und Gesundheit wird mit glänzenden, attraktiv aussehenden Haaren verbunden. Daher sind kosmetische Produkte, die speziell zur Erhöhung des Haarglanzes beitragen für den Verbraucher von großem Interesse.

Der Glanz ist eine Eigenschaft, die dadurch zustande kommt, dass die Oberfläche eines Stoffes so glatt ist, dass Vertiefungen kleiner sind als die Wellenlänge des sichtbaren Lichtes. Der Glanz keratinischer Fasern hängt somit von der Oberflächenbeschaffenheit ab. Je rauer die Oberfläche (Cuticula), desto größer ist der Anteil an diffus reflektierten Licht und desto geringer ist der Glanz der Faser.

Das Haar lässt sich grob in zwei wesentliche Bestandteile unterteilen: das Haarinnere (lat. Cortex) und die dachziegelförmig um das Innere gelegte äußere Umhüllung des Haares (lat. Cuticula).

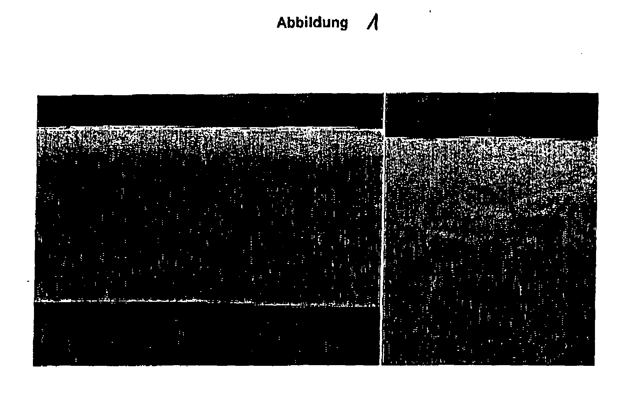

Die Cuticula besteht aus feinen, dachziegelartig geschichteten Schuppen, die verhornte Keratinozyten darstellen. Derart gleichmäßige Oberflächen findet man in der Regel an jungem Haar unmittelbar nach dem Austreten des Haares aus dem Haarschaft (siehe Abbildung 1).

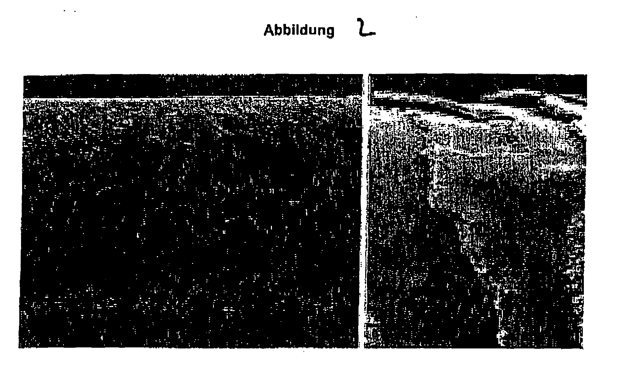

Die Cuticula bietet dem Cortex einerseits mechanischen und chemischen Schutz vor äußeren Einflüssen, andererseits Schutz gegen UV-Strahlen; sie verhindert das Austrocknen des Haares und die Entstehung von Spliss. Ferner ist die Oberfläche auch verantwortlich für das visuelle Erscheinungsbild, für den Glanz des Haares (siehe Abbildung 2).

Durch mechanische, chemische und umweltbedingte Einflüsse kommt es zur Veränderung und Schädigung des Haares, insbesondere der Cuticula. Die Cuticulaschuppen sind dann oft an den Enden abgebrochen und die einzelnen liegen nicht mehr eng an die Oberfläche an (siehe Abbildung 2). Solches Haar zeigt wenig Glanz.

Glanz keratinischer Fasern hängt folglich von der Glätte der Haaroberfläche ab. Zum Glätten der Haaroberfläche wurden bisher kosmetische Zubereitungen eingesetzt, in denen Öle, Wachse, Fette, Silikone oder pflegende Polymere eingesetzt werden, die eine hohe Affinität zum Haar haben und auf die Haaroberfläche aufziehen und diese dadurch glätten. Nachteil ist allerdings, dass diese Stoffe dem Haar oft ein fettiges Aussehen geben, es Beschweren und der damit erzielte Glanzeffekt höchstens bis zur nächsten Wäsche anhält. Pflegende Haarbehandlungsmittel werden beispielsweise in der WO 01/01934 A1, WO 00/61099, WO 2005107686 A1, US 20050158266 A1, EP 1569604 A1, US 20050112083 A1 beschrieben. In der DE 10058328 A1 wird z.B. ein Haarbehandlungsmittel mit einer quaternären Ammoniumverbindung und Fettalkoholen zur Haarglättung und Glanzerzielung beschrieben.

Darüber hinaus kann Haarglanz auch durch den Einsatz von Fruchtsäuren oder Panthenol erzielt werden. In der WO 1998/051264 A1 wird beispielsweise ein Haarreinigungsmittel mit glanzverbessernden Eigenschaften beschrieben, welches zu 5 bis 50 Gew.-% eines Tensids oder Tensidsgemisches, 2 bis 10 Gew.-% eines Gemisches von Fruchtsäuren und von 0,2 bis 2 Gew.-% Panthenol oder eines seiner Derivate enthält. In der DE 19504914 C1 wird ein Haarwaschmittel, enthaltend mindestens ein anionisches, nichtionisches und/oder zwitterionisches (amphoteres) Tensid und ein Gemisch aus Milchsäure, Citronensäure und Pyrrolidoncarbonsäure zur Verbesserung des Haarglanzes beschrieben.

Keine dieser Schriften offenbart aber erfindungsgemäße polyacrylathaltige Zubereitungen.

Gegenstand der hier vorliegenden Erfindung ist die Verwendung von Acrylaten (bekannt unter der Bezeichnung Carbopol) in kosmetischen Haarbehandlungsmitteln zur Erhöhung des Haarglanzes.

Carbopole® (Noveon) sind im Personal Care Bereich als hervorragende Verdicker, Rheologiemodifizier und Stabilisierer bekannt (siehe auch EP 1 723 945 A1). Die meisten Carbopol-Polymere sind hochmolekulare Homo- und Copolymere der Acrylsäure verbunden mit Polyalkenyl-Polyether. Gängigerweise werden sie im Personal Care Bereich in Konzentrationen von kleiner 1 % eingesetzt. Bekannt sind sie unter der INCl: Acrylates Copolymer, Polyacrylate-1 Crosspolymer, Acrylates/C10-30 Alkyl Acrylate Crosspolymer, Acrylates/C10-30 Alkyl Acrylate Crosspolymer und Carbomer. Im Hair Care Bereich werden Carbopole derzeit zur Stabilisierung von Silikonölen und Partikeln und als Verdicker eingesetzt.

Ganz überraschend wurde gefunden, dass ein Haarshampoo enthaltend Tensid und vernetzte Polymere auf Acrylatbasis, deren Gehalt an Tensiden 2 bis 25 Gew.-% beträgt, den Nachteilen des Standes der Technik abhilft. Die Einarbeitung von Carbopolen in solche Haarbehandlungsmittel, die keine Silikone enthalten, erhöht signifikant den Glanz keratinischer Fasern. Mittels Pyrolyse-GC/MS konnte nachgewiesen werden, dass Carbopol auf das Haar aufzieht (ca. 10 bis 500 µg/g Haar) und somit zu einer Glättung der Haaroberfläche führt. Es kann also auf Silikonöle verzichtet werden. Die erfindungsgemäßen Haarshampoos sind in Anspruch 1 definiert.

Der Gehalt an Tensiden beträgt 2 bis 25 Gew.-%. Die Einsatzkonzentration an vernetzten Polymeren auf Acrylatbasis beträgt 0.2 bis 0.3 Gew.-%.

Als vernetztes Polymere auf Acrylatbasis wird das Acrylates/C10-30 Alkyl Acrylate Crosspolymer (Carbopol ETD 2020) bei der Erhöhung des Haarglanzes verwendet.

Als Tenside können anionische, amphotere und nichtionische Tenside eingesetzt werden in Gesamtkonzentrationen von 2 bis 25 Gew.-%.

Besonders vorteilhaft ist es eine Kombination von Alkylethersulfaten (besonders Laurylethersulfat) und Cocamidopropylbetain, besonders bevorzugt Alkylethersulfate, Cocamidopropylbetain und Alkylpolyglykolestersulfosuccinate, ganz besonders bevorzugt Laurylethersulfat, Cocamidopropylbetain und Alkylpolyglykolestersulfosuccinate einzusetzen. Dabei ist es besonders bevorzugt, wenn zusätzlich Alkylpolyglykolestersulfosuccinate enthalten sind.

Besonders vorteilhaft ist Laurylethersulfat in Konzentrationen von 5 bis 15 Gew.-% und Cocamidopropylbetain in Konzentration von 0.5 bis 10 Gew.-% einzusetzen. Ganz besonders vorteilhaft ist es Laurylethersulfat in Konzentrationen von 6 bis 12 Gew.-% und Cocamidopropylbetain in Konzentrationen von 1 bis 5 Gew.-% einzusetzen.

Als ebenso sehr vorteilhaft hat sich die Kombination von Laurylethersulfat mit Cocamidopropylbetain und Decyl Glucosid erwiesen mit Konzentrationen von 2 bis 12 Gew.-% Laurylethersulfat, 2 bis 10 Gew.-% Cocamidopropylbetain und 2 bis 5 Gew.-% Decyl Glucosid.

Besonders bevorzugt ist es, wenn zusätzlich kationische Polymere enthalten sind.

Zur Pflege des Haares sind in dieser speziellen Zubereitung besonders vorteilhaft die Kombination der üblichen polymeren Haarkonditioniermittel (Polyquaternium-10, Guar Quats, PQ-7 und andere).

Besonders bevorzugt ist es, wenn zusätzlich Verdicker enthalten sind. Als Perlglanzsysteme können prinzipiell alle gängigen, bekannten Perlglanzrohstoffe verwendet werden. Besonders vorteilhaft ist die Verwendung von PEG-3 Distearat. Ganz besonders vorteilhaft ist es, wenn dieser Perlglanzrohstoff eine Teilchengröße aufweist in der Art, dass 90 % der Volumenanteile eine Teilchengröße von kleiner 50µm aufweisen. Die Einsatzkonzentrationen des Perlglanzes liegen vorteilhafterweise bei 5 bis 15 Gew.-%.

In den erfindungsgemäßen Haarshampoos ist zusätzlich Diamant enthalten.

Die Kombination von Diamant und Carbopol erhöht zusätzlich den Glanz des Haars. Diamant ist eine der kristallisierten Modifikationen des Kohlenstoffs. Er ist bei Oberflächenbedingungen gegenüber Graphit eigentlich thermodynamisch metastabil. Diamant hat eine Dichte von 3,52 t/m³ und eine Mohs-Härte von 10, er ist damit das härteste irdische Material. Diamanten können im Stahlmörser zu Pulver zerstampft werden.

Diamanten sind als hochwertige Schmucksteine bekannt. Eine höhere wirtschaftliche Bedeutung haben sie aber heute durch ihre industrielle Verwendung als Schneidstoffe und Schleifstoffe sowie als Zugabe in Polierpasten, wobei man sich ihre Härte und Verschleißfestigkeit zunutze macht.

Diamanten können auch synthetisch hergestellt werden. Dies gelang erstmals 1953/55. Die heutigen im thermodynamischen Stabilitätsbereich von Diamant operierenden Hochtemperatur-Hochdruck-Verfahren basieren auf der Umwandlung von Graphit in Diamant bei 1200 bis 1400°C und 5 bis 7 GPa Druck unter Mitwirkung geschmolzener Metalle als Lösemittel und Katalysatoren, meist Eisen, Cobalt, Chrom, Aluminium und Titan, früher auch Nickel. Dabei können durch Optimierung der Züchtungsbedingungen wie chemische Reinheit des eingesetzten Kohlenstoffs (z. B. ¹²C) und geeignete Zusammensetzungen der metallischen Flussmittel Stickstoff-arme Kristalle mit verbesserten optischen Eigenschaften hergestellt werden. 1990 wurde ein synthetischer Diamantkristall von 14,2 Karat Gewicht hergestellt. Industriell übliche Synthesen erfolgen mit dem Kohlenstoff-Isotop ¹²C. Seit 1991 können farblose, lupenreine Diamanten mit 3 Karat Gewicht aus 99 %¹³C hergestellt werden.

Mit dem Verfahren der CVD-Synthese (englisch: chemical vapour deposition) lassen sich Diamanten auch bei Atmosphärendruck aus ∼1000°C heißen Gasen (Methan oder andere Kohlenwasserstoffe) in Gegenwart eines Wasserstoff-Überschusses abscheiden. Dabei entsteht ein nanometer- bis mikrometerdünner metastabiler Diamantfilm auf Metallsubstraten.

Nanokristalline Diamanten können durch intensive Bestrahlung von Graphit erhalten werden. Dabei entstehen zunächst zwiebelartige Schalenstrukturen aus Kohlenstoff-Atomen, die bei fortgesetzter Bestrahlung Bindungen zwischen den Schalen bilden und damit eine Selbstkompression verursachen, bei der Diamantkristallite gebildet werden. Mit einer laserbeheizten transparenten Diamantstempelzelle ist es heute möglich, die Druck-Temperatur-Bedingungen im Erdkern zu simulieren.

In den erfindungsgemäßen Haarshampoos liegt Diamant als Pulver vor in einer Konzentrationvon 0,0001 bis 1 Gew.-%, bevorzugt 0,001 bis 1 Gew.%, besonders bevorzugt 0,005 bis 0,1 Gew.-% beträgt.

Die mittlere Teilchengröße der Diamantpartikel 1 bis 20 µm, besonders bevorzugt 4 bis 6 µm .

Es hat sich ebenfalls herausgestellt, das der erfindungsgemäße Einsatz von Polyacrylaten die Einarbeitung von Diamantpulver ermöglicht. Damit gelingt es besonders gut, die Diamantpartikel Partikel in der Schwebe zu halten, so dass diese nicht absinken.

Verwendet werden können Diamanten verschiedener Partikelgröße. Als vorteilhaft haben sich Diamanten mit einer Teilchengröße von 1 bis 20 µm erwiesen, so genannte Diamant-Mikrokörnungen (erhältlich bei Firma Microdiamant). Als ganz besonders vorteilhaft hat sich eine mittlerer Teilchengröße von 4 bis 6 µm gezeigt. Als bereits sehr wirkungsvoll haben sich Konzentrationen im Bereich von 0.001 von 1 Gew.-% gezeigt.

Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten werden.

Die Erfindung umfasst auch die Verwendung eines der beschriebenen Haarshampoos zur Verbesserung des Glanzes von Haaren.

### Beispiele:

Eingesetzt wurde Diamantpulver erhältlich bei Firma Microdiamant, Partikelgröße von 2,85 bis 18 µm, mittlere Partikelgröße 5 µm.
Nicht erfindungsgemäße Beispiele sind mit einem Stern gekennzeichnet.

### 1) Conditioner-Shampoo

| | **1*** | **2** | **3** | **4** | **5** | **6*** | **7*** |
|---|---|---|---|---|---|---|---|
| Natrium Laurylethersulfat | 10 | 9 | 8 | 9 | 9 | 10 | 12 |
| Cocamidopropyl Betain | 1.5 | - | 3.5 | 3.5 | 4 | 4 | 3 |
| Di-Natrium PEG-5 Laurylcitrat Sulfosuc cinat | - | - | 2 | 3 | 3 | - | - |
| Kokamid DEA | 3 | | | | | | |
| Decyl Glucoside | - | - | 3 | - | - | - | - |
| Natrium Cocoamphoacetat | - | 3 | - | - | - | - | - |
| Polyquaternium-10 | 0.1 | - | - | 0.3 | 0.3 | 0.2 | 0.1 |
| Polyquaternium-7 | - | - | 0.2 | 0.2 | - | - | 0.2 |
| Guar Hydroxypropyltrimoniumchlorid | 0.05 | 0.3 | 0.1 | - | 0.1 | - | - |
| Acrylates/C10-30 Alkyl Acrylate Cross-polymer | 0.1 | 0.2 | 0.2 | 0.3 | 0.3 | 0.5 | 1 |
| PEG-200 Hydrogenated Glyceryl Palmate | - | - | 0,2 | - | - | 1,0 | - |
| PEG-40 hydrogeniertes Rizinusöl | 0.2 | 0.3 | 0.4 | 0,5 | 0.6 | 0,5 | 0.5 |
| PEG-3 Distearat | 1.5 | - | 0.75 | 1.5 | 1.0 | - | 0.5 |
| Glycol Distearat | - | 4.0 | - | - | - | - | - |
| PEG-7 Glycerylcocoat | 0.5 | 1 | 2 | - | - | - | - |
| Natrium Salicylat | 0.2 | 0.3 | 0.4 | 0.4 | 0.4 | - | - |
| Natrium Benzoat | 0.5 | 0.4 | 0.3 | 0.2 | 0.4 | - | 0.5 |
| Parfüm | q.s. | q.s. | q.s. | q.s. | q.s. | q.s. | - |
| Benzophenon-4 | 0,2 | 0,25 | - | 0,3 | - | - | - |
| Oryzanol | - | - | - | 0,5 | - | - | 0,05 |
| Piroctone Olamine | - | - | - | - | - | 0.1 | 0.2 |
| Diamond powder | 0.005 | 0.01 | 0.001 | 0.002 | 0.01 | 0.05 | 0.1 |
| Niacinamid | - | 0.01 | - | - | - | - | - |
| Panthenol | - | - | 0.01 | - | - | - | 0.01 |
| Jojobaöl | - | - | - | - | 0,1 | - | - |
| White Calla | 0.001 | 0.01 | 0.1 | 0.2 | 0.5 | 0.005 | 0.02 |
| Mica | 0.05 | - | 0.01 | 0.03 | - | 0.05 | - |
| Natronlauge | q.s. | q.s. | q.s. | q.s. | q.s. | q.s. | - |
| Citronensäure | q.s. | q.s. | q.s. | q.s. | q.s. | q.s. | q.s. |
| Wasser | ad 100 | ad 100 | ad 100 | ad 100 | ad 100 | ad 100 | ad 100 |

### 2) Conditioner-Shampoo mit Perlglanz

| | **1*** | **2** | **3** | **4** | **5*** | **6*** |
|---|---|---|---|---|---|---|
| Natrium Laurylethersulfat | 10 | 9 | 9 | 9 | 10 | 12 |
| Cocamidopropyl Betain | 1.5 | 3 | 4 | 3 | 4 | 3 |
| Dinatrium PEG-5 Laurylcitrat Sulfosuccinat | - | 3 | 3 | 2 | - | 4 |
| Kokamid DEA | 3 | - | - | - | - | - |
| PEG-200 Hydrogenated Glyce ryl Palmate | - | - | 0.1 | - | 0,3 | 0,4 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimonium-chlorid | 0.05 | 0.3 | 0.1 | - | 0.1 | - |
| Acrylates/C10-30 Alkyl Acry late Crosspolymer | 0.1 | 0.2 | 0.3 | 0.3 | 0.5 | 1.0 |
| PEG-3 Distearat | 1.5 | 3 | 4 | 2 | 2,5 | 0,75 |
| Glycol Distearat | - | - | - | - | 0.5 | 0,5 |
| PEG-40 hydriertes Rizinusöl | 0.2 | 0.3 | 0.4 | 0.5 | 0.2 | 0,3 |
| Diamond powder | 0.005 | 0.01 | 0.001 | 0.1 | 0.01 | 0.05 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| White Calla | 0.001 | 0.01 | 0.1 | 0.2 | 0.5 | 0.005 |
| Mica | 0.05 | - | 0.01 | 0.03 | - | 0.05 |
| Natronlauge | q.s. | q.s. | q.s. | q.s. | q.s. | q.s. |
| Zitronensäure | q.s. | q.s. | q.s. | q.s. | q.s. | q.s. |
| Wasser | ad 100 | ad 100 | ad 100 | ad 100 | ad 100 | ad 100 |

### 3) Klares Conditioning-Shampoo

| | **1*** | **2** | **3** | **4** | **5*** | **6*** |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 1.5 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Cocamide DEA | 3 | | | | | |
| Decylglucosid | - | - | - | 2 | 4.5 | - |
| PEG-200 Hydrogenated Glyce ryl Palmate | 0.1 | - | - | 0.4 | 0.5 | - |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimonium-chlorid | 0.05 | 0.3 | 0.1 | - | 0.1 | - |
| Acrylates/C10-30 Alkyl Acry late Crosspolymer | 0.1 | 0.2 | 0.3 | 0.3 | 0.5 | 1.0 |
| Diamond powder | 0.005 | 0.01 | 0.001 | 0.1 | 0.01 | 0.05 |
| Hydrolysiertes Seidenprotein | - | 0,1 | 0,2 | 0,3 | 0.3 | - |
| White Calla | 0.001 | 0.01 | 0.1 | 0.2 | 0.5 | 0.005 |
| Mica | 0.05 | - | 0.01 | 0.03 | - | 0.05 |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 4) Mildes Baby Shampoo

| | **1*** | **2** | **3** | **4** | **5*** | **6*** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyce ryl Palmate | - | - | - | 0.2 | 0.3 | 0,4 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimonium-chlorid | 0.05 | 0.3 | 0.1 | - | 0.1 | - |
| Acrylates/C10-30 Alkyl Acry late Crosspolymer | 0.1 | 0.2 | 0.3 | 0.3 | 0.5 | 1.0 |
| Diamond powder | 0.005 | 0.01 | 0.001 | 0.1 | 0.01 | 0.05 |
| PEG-3 Distearat | - | - | 0,5 | 2 | - | - |
| White Calla | 0.001 | 0.01 | 0.1 | 0.2 | 0.5 | 0.005 |
| Mica | 0.05 | - | 0.01 | 0.03 | - | 0.05 |
| Glycol Distearat | - | - | - | - | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 5) Antischuppen Shampoo/Mildes Kopfhaut Shampoo (Formel 5)

| | **1*** | **2** | **3** | **4** | **5*** | **6*** |
|---|---|---|---|---|---|---|
| Natrium Laurylethersulfat | 10 | 9 | 9 | 9 | 10 | - |
| Natrium Myristylethersulfat | - | - | - | - | - | 6 |
| Cocamidopropyl Betain | 1.5 4 | | 3 | 3 | 4 | 5 |
| Dinatrium PEG-5 Laurylcitrat Sulfosuccinat | - | 3 | 3 | 3 | - | - |
| Natrium Cocoamphoacetat | - | - | - | - | - | 2.5 |
| Cocamide DEA | 3 | - | - | - | - | - |
| Decylglucosid | - | - | - | - | - | 2.5 |
| PEG-200 Hydrogenated Glyce ryl Palmate | - | - | 0.1 | - | 0.4 | 3 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimonium-chlorid | 0.05 | 0.3 | 0.1 | - | 0.1 | - |
| Acrylates/C10-30 Alkyl Acry late Crosspolymer | 0.1 | 0.2 | 0.3 | 0.3 | 0.5 | 1.0 |
| Diamond powder | 0.005 | 0.01 | 0.001 | 0.1 | 0.01 | 0.05 |
| Climbazol | 0.5 | 0.5 | - | 0.5 | 1.0 | - |
| White Calla | 0.001 | 0.01 | 0.1 | 0.2 | 0.5 | 0.005 |
| Mica | 0.05 | - | 0.01 | 0.03 | - | 0.05 |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| PEG-3 Distearat | 1.5 | 3 | 0,75 | 0,5 | - | - |
| Glycol Distearat | - | - | - | - | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

## Patentansprüche

1. Haarshampoo enthaltend 2 bis 25 Gew.% Tenside und 0,2 bis 0,3 Gew.% vernetzte Polymere auf Acrylatbasis, wobei als vernetztes Polymer auf Acrylatbasis Acrylates/C10-30 Alkyl Acrylate Crosspolymer enthalten ist **dadurch gekennzeichnet, dass** zusätzlich 0,0001 bis 1 Gew.% Diamantpulver mit einer mittleren Teilchengröße von 1 bis 20 µm enthalten sind.

2. Haarshampoo nach Patentanspruch 1 **dadurch gekennzeichnet, dass** als Tenside anionische, amphotere oder nichtionische Tenside enthalten sind.

3. Haarshampoo nach einem der vorangehenden patentansprüche **dadurch gekennzeichnet, dass** als Tenside Alkylethersulfate und Cocoamidopropylbetain enthalten sind.

4. Haarshampoo nach Patentanspruch 3 **dadurch gekennzeichnet, dass** 5 bis 15 Gew.°% Laurylethersulfat und 0,5 bis 10 Gew.% Cocoamidopropylbetain enthalten sind.

5. Haarshampoo nach einem der vorangehenden Patentansprüche **dadurch gekennzeichnet, dass** zusätzlich kationische Polymere enthalten sind.

6. Verwendung eines Haarshampoos nach einem der vorangehenden Patentansprüche zur Verbesserung des Glanzes von Haaren.

## Claims

1. Hair shampoo comprising 2 to 25% by weight of surfactants and 0.2 to 0.3% by weight of crosslinked acrylate-based polymers, where the crosslinked acrylate-based polymer present is Acrylates/C10-30 Alkyl Acrylate Crosspolymer, **characterized in that** additionally 0.0001 to 1% by weight of diamond powder with an average particle size of from 1 to 20 µm are present.

2. Hair shampoo according to Patent Claim 1, **characterized in that** the surfactants present are anionic, amphoteric or nonionic surfactants.

3. Hair shampoo according to one of the preceding patent claims, **characterized in that** the surfactants present are alkyl ether sulphates and cocoamidopropylbetaine.

4. Hair shampoo according to Patent Claim 3, **characterized in that** 5 to 15% by weight of lauryl ether sulphate and 0.5 to 10% by weight of cocoamidopropylbetaine are present.

5. Hair shampoo according to one of the preceding patent claims, **characterized in that** cationic polymers are additionally present.

6. Use of a hair shampoo according to one of the preceding patent claims for improving the shine of hair.

## Revendications

1. Shampooing capillaire contenant 2 à 25 % en poids de tensioactifs et 0,2 à 0,3 % en poids de polymères réticulés à base d'acrylate, en tant que polymère réticulé à base d'acrylate étant contenu de l'acrylates/C10-30 alkyl acrylate crosspolymer, **caractérisé en ce qu'**en outre est contenu 0,0001 à 1 % en poids de poudre de diamant ayant une taille moyenne de particule de 1 à 20 µm.

2. Shampooing capillaire selon la revendication 1, **caractérisé en ce que** comme tensioactifs sont contenus des tensioactifs anioniques, amphotères ou non ioniques.

3. Shampooing capillaire selon l'une quelconque des revendications précédentes, **caractérisé en ce que** comme tensioactifs sont contenus des alkyléthersulfates et de la cocosamidopropylbétaïne.

4. Shampooing capillaire selon la revendication 3, **caractérisé en ce que** sont contenus 5 à 15 % en poids de lauryléthersulfate et 0,5 à 10 % en poids de cocosamidopropylbétaïne.

5. Shampooing capillaire selon l'une quelconque des revendications précédentes, **caractérisé en ce que** des polymères cationiques sont en outre contenus.

6. Utilisation d'un shampooing capillaire selon l'une quelconque des revendications précédentes, pour l'amélioration du brillant des cheveux.
